# EUROPEAN PATENT SPECIFICATION

(11) **EP 2 207 218 B1**
(45) Date of publication and mention of the grant of the patent: **15.08.2012**
(21) Application number: 08831476.0
(22) Date of filing: 18.09.2008
(51) Int. Cl.: H01L 51/00, C07D 495/04, C07D 517/04

(54) **FIELD EFFECT TRANSISTOR**
FELDEFFEKTTRANSISTOR
TRANSISTOR A EFFET DE CHAMP

(30) Priority: 21.09.2007 JP 2007245460
(43) Date of publication of application: 14.07.2010
(73) Proprietor: Nippon Kayaku Kabushiki Kaisha, Tokyo 102-8172 (JP); Hiroshima University, Hiroshima 739-8511 (JP)
(72) Inventor: KUWABARA, Hirokazu, Tokyo 115-8588 (JP); YUI, Tatuto, Tokyo 115-8588 (JP); IKEDA, Masaaki, Tokyo 115-8588 (JP); TAKIMIYA, Kazuo, Higashihiroshima-shi Hiroshima 739-8527 (JP)
(74) Representative: Gille Hrabal
(86) International application number: PCT/JP2008/066842
(87) International publication number: WO 2009/038120

(56) References cited:
- EP-A1- 1 357 163
- EP-A1- 2 147 923
- JP-A- 2005 154 371
- JP-A- 2005 330 185
- KAZUO TAKIMIYA ET AL.: 'Molecular Modification of 2,6-Diphenylbenzo[1,2-b:4,5-b'] dichalcogenophenes by Introduction of Strong Electron-withdrawing Groups: Conversion from p- to n-Channel OFET Materials' CHEMISTRY LETTERS vol. 35, no. 10, 05 October 2006, pages 1200 - 1201, XP009142643

## Description

### TECHNICAL FIELD

The present invention relates to a field effect transistor. More specifically, the present invention relates to a field effect transistor characterized by having a semiconductor layer made of a specific organic heterocyclic compound.

### BACKGROUND ART

A field effect transistor is a device generally having a semiconductor layer (a semiconductor film), a source electrode, a drain electrode, a gate electrode formed in opposite to these electrodes via an insulating layer, on a substrate, and others. A field effect transistor has been widely used not only in integrated circuits as a logical circuit element but also as a switching element or the like. A semiconductor layer is usually made of a semiconductor material. At present, inorganic semiconductor materials mainly containing silicon have been used in field effect transistors. Particularly, a thin-film transistor in which a semiconductor layer from amorphous silicon is formed on a substrate of glass or the like has been used in displays and others. When such inorganic semiconductor materials are used in manufacturing field effect transistors, they have to be treated at high temperature or in vacuum. Accordingly, high investment for equipments and much energy are required for manufacturing them, and thereby the costs are increased very much. In addition, these materials are exposed to high temperature in manufacturing field effect transistors. Therefore, materials with an insufficient heat resistance, e.g., a film or a plastic, cannot be used for a substrate. As a result, a flexible material, which can be bent, folded or the like, cannot be used for a substrate, whereby the applications thereof are limited.

On the other hand, researches and developments have been made on field effect transistors manufactured by using an organic semiconductor material. Use of an organic material makes it possible to manufacture them in a low-temperature process, requiring no high temperature treatment, and extend the range of materials that can be used for a substrate. As a result, it becomes realizable to manufacture field effect transistors more flexible, lighter and more irrefrangible than conventional ones. Furthermore, in a process of manufacturing field effect transistors, a method of applying a solution of a semiconductor material and a printing method with inkjet or the like can be employed. Therefore, large-area field effect transistors can be manufactured at low cost. Moreover, various types of compounds can be selected for an organic semiconductor material. Thus, based on their characteristics, it has been being expected to develop novel functions which have not been ever found.

Various studies have been conducted so far on use of an organic compound as a semiconductor material. For example, semiconductor materials from a pentacene, a thiophene, or an oligomer or a polymer thereof have been already known as ones having hole transport characteristics (see Patent Documents 1 and 2). A pentacene is an acene-type aromatic hydrocarbon having 5 linearly-fused benzene rings. A field effect transistor using a pentacene as a semiconductor material has been reported to have equivalent charge mobility (carrier mobility) to that of amorphous silicon in practical use.

However, the characteristics of a pentacene are largely affected by the purity, but it is difficult to purify it. In addition, a pentacene is expensive to manufacture. Thus, it has many problems to be used as a transistor material. Furthermore, field effect transistors using the compound are environmentally-degradable and questioned in stability. The same problems are also caused when a thiophene compound is used. Accordingly, neither of the compounds would be highly useful in practice.

In the meantime, as a liquid crystalline compound, an alkyl derivative (8 to 12 carbon atoms) and an alkoxy derivative (8 to 12 carbon atoms) of 2,6-diphenylbenzo[[1,2-b:4,5-b']]dithiophene have been studied (see Patent Document 3). Furthermore, as an organic semiconductor material, 2,6-diphenylbenzo[[1,2-b:4,5-b']]diselenophene, 2,6-bis[(4,1'-biphenyl)-1-yl]-benzo[1,2-b:4,5-b']diselenophene and the like have been studied. They have relatively high electron mobility and stability even in the atmosphere, whereby they have been expected to be put into pratical use (see Patent Document 4 and Non-Patent Documents 1, 2).

However, these compounds have a relatively high threshold voltage. It is thus matters of concern in practical use that a high driving voltage is required, thereby causing increased power consumption. For instance, in case of a mobile display driven by a rechargeable battery, the high threshold voltage causes a reduced operation time. In addition, the high threshold voltage decreases the operation speed. Because of these problems, it has been desired to reduce the threshold voltage.
In addition, these compounds do not have sufficient durability in terms of heat resistance and wet heat resistance. More specifically, the carrier mobility rapidly decreases in relatively high temperature or humid environment. It has been also desired to improve these problems.
Moreover, it has been investigated what is affected by substrate temperature upon the characteristics of a semiconductor layer, in particular, carrier mobility and a threshold voltage, when a semiconductor layer from a standard organic semiconductor material such as a pentacene is formed on a substrate.
Patent Document 1: JP 2001-94107 A
Patent Document 2: JP 6-177380 A
Patent Document 3: JP 2005-330185 A
Patent Document 4: JP 2005-154371 A
Non-Patent Document 1: J. Am. Chem. Soc., Vol. 126, 5084-5085 (2004)
Non-Patent Document 2: Chem. Letters, Vol. 35, No. 10, 1200-1201 (2006)
Prior art document EP 2 147 923 A1 which is a prior art document under Article 54(3) EPC discloses the two following compounds:

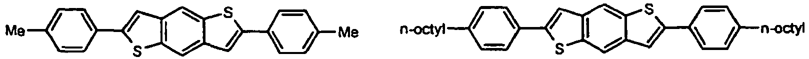

### DISCLOSURE OF THE INVENTION

### PROBLEMS TO BE SOLVED BY THE INVENTION

An object of the present invention is to provide an organic compound having excellent carrier mobility, excellent heat resistance and humidity resistance, a practical threshold voltage and other characteristics; a semiconductor material from the compound; and a field effect transistor having a semiconductor layer made of the compound.

### MEANS FOR SOLVING THE PROBLEMS

The present inventors conducted studies with a view to solving the aforementioned problems, and have found that an alkyl derivative of 2,6-diphenyl benzo[1,2-b:4,5-b']dichalcogenophene having a specific structure has excellent characteristics compared to conventional organic semiconductor materials in respect to the aforementioned problems. The present invention has been accomplished based on these findings.

Thus, according to an aspect of the present invention, there are provided
(1) A field effect transistor characterized in that a semiconductor layer is formed by use of at least one compound represented by the following Formula (1) as a semiconductor material, 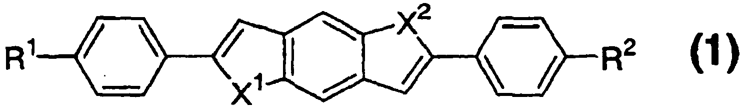 wherein, X¹ and X² each independently represent a sulfur atom, a selenium atom or a tellurium atom and R¹ and R² each independently represent a saturated or unsaturated linear, branched or cyclic C1-C14 alkyl group,
   with the proviso that, when both of X¹ and X² are a selenium atom, R¹ and R² each independently represent a saturated or unsaturated linear, branched or cyclic C1-C4 alkyl group.

The field effect transistors according to preferable embodiments of the present invention include:
a field effect transistor as described above, further characterized by a compound represented by Formula (1) wherein R¹ and R² are each independently a saturated or unsaturated linear, branched or cyclic C1-C8 alkyl group, with the proviso that, when both of X¹ and X² are a selenium atom, R¹ and R² each independently represent a saturated or unsaturated linear, branched or cyclic C1-C4 alkyl group;
a field effect transistor as described in any one of the above items, further characterized by a compound represented by Formula (1) wherein X¹ and X² are the same and represent a sulfur atom or a selenium atom, and R¹ and R² are each independently a saturated or unsaturated linear, branched or cyclic C1-C4 alkyl group;
a field effect transistor described in any one of the above items, further characterized by a compound represented by Formula (1) wherein X¹ and X² are the same and represent a sulfur atom or a selenium atom, and R¹ and R² are each independently a linear C1-C4 alkyl group;
a field effect transistor described in any one of the above items, further characterized by a compound represented by Formula (1) wherein X¹ and X² are the same and represent a sulfur atom or a selenium atom, and R¹ and R² are the same and represent an unsubstituted linear C1-C4 alkyl group;
a field effect transistor described in any one of the above items, further characterized by a compound represented by Formula (1) wherein X¹ and X² are the same and represent a sulfur atom or a selenium atom, and R¹ and R² are the same and represent a methyl group or an ethyl group;
a field effect transistor described in any one of the above items, further characterized by a compound represented by Formula (1) wherein X¹ and X² are the same and represent a sulfur atom or a selenium atom, and R¹ and R² are the same and represent a methyl group;
a field effect transistor described in any one of the above items, further characterized in that the semiconductor layer has a single-layer structure; and
a field effect transistor described in any one of the above items, further characterized in that the semiconductor layer has a single-layer structure made of a single compound represented by Formula (1).

Furthermore, according to another aspect of the present invention, there is provided
a compound represented by the following Formula (2): 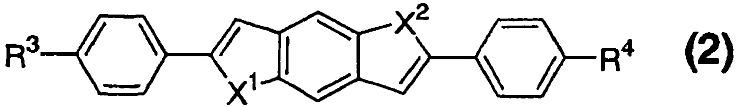 wherein, X¹ and X² each independently represent a sulfur atom, a selenium atom or a tellurium atom, and R³ and R⁴ each independently represent a saturated or unsaturated linear, branched or cyclic C1-C4 alkyl group, with the proviso, that when both of x¹ and X² are a sulfur atom, R³ and R⁴ are not both a methyl group.

The compounds according to preferable embodiments of the present invention include:
a compound as described above, in which X¹ and X² in Formula (2) are the same and represent a sulfur atom or a selenium atom, and R³ and R⁴ are the same and represent an unsubstituted linear C1-C4 alkyl group with the proviso that when both of X¹ and X² are a sulfur atom, R³ and R⁴ are not both a methyl group ; and
a compound as described above, in which X¹ and X² in Formula (2) are the same and represent a sulfur atom or a selenium atom, and R³ and R⁴ are the same and represent an ethyl group.

### ADVANTAGES OF THE INVENTION

By means of a specific alkyl derivative of a specific 2,6-diphenylbenzo[1,2-b:4,5-b']dichalcogenophene represented by Formula (1), an organic field effect transistor having excellent carrier mobility, excellent stability with respect to heat resistance, wet heat resistance and the like, and a lower threshold voltage than a conventional one, can be provided.

### BRIEF DESCRIPTION OF THE DRAWINGS

Fig. 1 shows schematic views illustrating the structures of field effect transistors according to embodiments of the present invention.
Fig. 2 shows schematic views illustrating the steps of manufacturing a field effect transistor according to an embodiment of the present invention.
Fig. 3 shows a schematic view of the field effect transistor obtained in Example 1 according to the present invention.

### DESCRIPTION OF REFERENCE NUMERALS

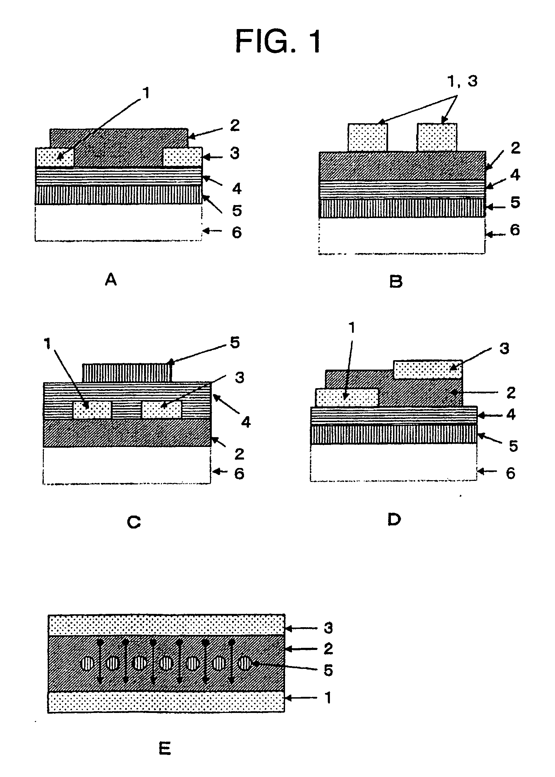

The same reference numbers as those below are used to designate the same structural elements as those below in Fig. 1 to Fig. 3.
1. Source electrode
2. Semiconductor layer
3. Drain electrode
4. Insulating layer
5. Gate electrode
6. Substrate
7. Protective layer

### BEST MODE FOR CARRYING OUT THE INVENTION

The present invention will be more specifically described.
It is a first embodiment of the present invention to provide an organic field effect transistor using a specific organic compound as a semiconductor material. Specifically, it relates to an organic field effect transistor of which semiconductor layer is formed by using a compound represented by Formula (1) as the semiconductor material. Then, first, compounds of Formula (1), as mentioned above, will be described.

In Formula (1), X¹ and X² each independently represent a sulfur atom, a selenium atom or a tellurium atom, and R¹ and R² each independently represent a saturated or unsaturated linear, branched or cyclic C1-C14 alkyl group,
with the proviso that when both of X¹ and X² are a selenium atom, R¹ and R² each independently represent a saturated or unsaturated linear, branched or cyclic C1-C4 alkyl group.

X¹ and X² are each independently a sulfur atom, a selenium atom or a tellurium atom, preferably a sulfur atom or a selenium atom, and particularly preferably a sulfur atom. Furthermore, X¹ and X² are more preferably the same, and further preferably the same and represent a sulfur atom or a selenium atom. When they are the same, they particularly preferably represent a sulfur atom.

An alkyl group represented by R¹ and R² includes a linear, branched or cyclic alkyl group, and the number of carbon atoms thereof is 1 to 14, preferably 1 to 8, more preferably 1 to 4, and further preferably 1 to 3.
Examples of a saturated linear alkyl group include methyl, ethyl, propyl, n-butyl, n-pentyl, n-hexyl, n-octyl, n-decyl, n-dodecyl and tetradecyl.
Examples of a saturated branched alkyl group include isopropyl, isobutyl and t-butyl.
Examples of a saturated cyclic alkyl group include cycloalkyl groups having carbon atoms of 3 to 14 such as cyclohexyl, cyclopentyl, adamantyl and norbornyl.
Examples of an unsaturated alkyl group include allyl.
As a C1-C14 alkyl group, a saturated alkyl group is preferred to an unsaturated alkyl group, and an unsubstituted alkyl group is preferred to a substituted alkyl group.
A C1-C8 saturated linear alkyl group is more preferable, a C1-C4 saturated linear alkyl group is further preferable, methyl or ethyl is particularly preferable, and methyl is most preferable.
R¹ and R² each independently represent an alkyl group as mentioned above and may be the same or different and more preferably the same.
When both of X¹ and X² are a selenium atom, R¹ and R² each independently represent a C1-C4 alkyl group. In this case, the "C1-C4 alkyl group" is preferably a C1-C2 alkyl group, and particularly preferably a methyl group.

A compound having a combination of atoms and groups mentioned as preferable examples of X¹, X², R¹ and R² in the above is more preferable, a compound having a combination of atoms and groups mentioned as more preferable examples is further preferable, and a compound having a combination of atoms and groups mentioned as particularly preferable examples is particularly preferable.

It is a further embodiment of the present invention to provide novel compounds represented by the following Formula (2): 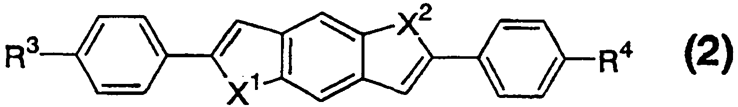 wherein, X¹ and X² each independently represent a sulfur atom, a selenium atom or a tellurium atom, and R³ and R⁴ each independently represent a saturated or unsaturated linear, branched or cyclic C1-C4 alkyl group.

In Formula (2), X¹ and X² are defined as the same as those in Formula (1) including preferable examples.

R³ and R⁴ may be the same as a saturated or unsaturated linear, branched or cyclic C1-C4 alkyl of the groups identified as R¹ and R² in Formula (1), and preferable groups thereof may be also the same as the ones identified as such groups in R¹ and R².
When both of X¹ and X² are a sulfur atom, R³ and R⁴ are not both a methyl group.

Also in Formula (2), a compound having a combination of atoms and groups mentioned above as preferable examples of X¹, X², R¹ and R² is more preferable, a compound having a combination of atoms and groups mentioned above as more preferable examples is further preferable, and a compound having a combination of atoms and groups mentioned above as particularly preferable examples is particularly preferable.

A compound represented by Formula (1) can be synthesized by known methods disclosed in Non-Patent Documents 1 and 2 or others. For example, as shown in the following Scheme 1, a compound of interest can be easily obtained with high purity by the two steps of: obtaining a compound of Formula (B) from 1,4-dibromo-2,5-diiodobenzene represented by Formula (A) and obtaining a compound of interest from a compound of Formula (B). More specifically, for example, according to the methods described in Patent Document 4 and Non-Patent Document 3, compound (A) can be reacted with an aryl acetylene derivative in diisopropylamine in the presence of palladium chloride bis(triphenylphosphine) copper iodide to obtain compound (B). Further, compound (B) is reacted with a sulfur compound such as sodium sulfate in an amide solvent such as N-methylpyrrolidone at about 140°C for about 8 hours to obtain a compound represented by Formula (1).

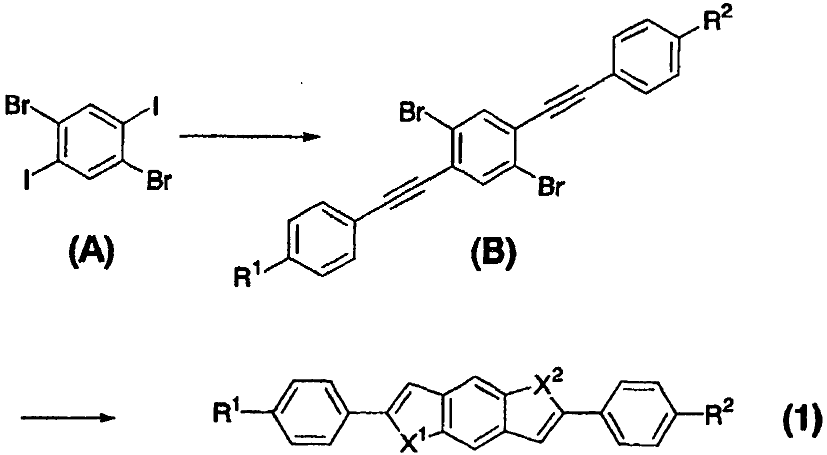

A method for purifying a compound represented by Formula (1), as mentioned above, not being particularly limited, includes known methods such as recrystallization, column chromatography and vacuum sublimation purification. If necessary, these methods may be used in combination.

Examples of a compound represented by Formula (1), as mentioned above, are shown in the following Table 1.

**[Table 1-1]**

| Table 1 | | | | |
|---|---|---|---|---|
| Compound No. | X1 | X2 | R1 | R2 |
| 1 (not part of the invention) | S | S | CH3 | CH3 |
| 2 | S | S | C2H5 | C2H5 |
| 3 | S | S | i-C3H7 | 1-C3H7 |
| 4 | S | S | n-C3H7 | n-C3H7 |
| 5 | S | S | n-C4H9 | n-C4H9 |
| 6 | S | S | t-C4H9 | t-C4H9 |
| 7 | S | S | i-C4H9 | i-C4H9 |
| 8 | S | S | n-C5H11 | n-C5H11 |
| 9 | S | S | n-C6H13 | n-C6H13 |
| 10 | S | S | n-C7H15 | n-C7H15 |
| 11 (not part of the invention) | S | S | n-C8H17 | n-C8H17 |
| 12 (not part of the invention) | **S** | **S** | n-C9H19 | n-C9H19 |
| 13 (not part of the invention) | S | S | n-C10H21 | n-C18H21 |
| 14 (not part of the invention) | S | S | n-C11H23 | n-C11H23 |
| 15 (not part of the invention) | S | S | n-C12H25 | n-C12H25 |
| 16 (not part of the invention) | S | S | n-C13H27 | n-C13H27 |
| 17 (not part of the invention) | S | S | n-C14H29 | n-C14H29 |
| 18 | S | S | CH3 | n-C8H17 |
| 19 | S | S | CH3 | n-C12H25 |
| 20 | S | S | C2H5 | CH3 |
| 21 | Se | Se | CH3 | CH3 |
| 22 | Se | Se | C2H5 | C2H5 |
| 23 | Se | Se | i-C3H7 | i-C3H7 |
| 24 | Se | Se | n-C3H7 | n-C3H7 |
| 25 | Se | Se | n-C4H9 | n-C4H9 |
| 26 | Se | Se | t-C4H9 | t-C4H9 |
| 27 | Se | Se | i-C4H9 | i-C4H9 |
| 28 (not part of the invention) | Se | Se | n-C5H11 | n-C5H11 |
| 29 (not part of the invention) | Se | Se | n-C6H13 | n-C6H13 |
| 30 (not part of the invention) | Se | Se | n-C7H15 | n-C7H15 |
| 31 (not part of the invention) | Se | Se | n-C5H17 | n-C8H17 |
| 32 (not part of the invention) | Se | Se | n-C9H19 | n-C9H19 |
| 33 (not part of the invention) | Se | Se | n-C10H21 | n-C10H21 |

| | | | | |
|---|---|---|---|---|
| * (not part of the invention) | | | | |

**[Table 1-2]**

| Table 1 (continued) | | | | |
|---|---|---|---|---|
| Compound No. | X1 | X2 | R1 | R2 |
| 34 (not part of invention) | Se | Se | n-C11H23 | n-C11H23 |
| 35 (not part of invention) | Se | Se | n-C12H25 | n-C12H25 |
| 36 (not part of invention) | Se | Se | n-C13H27 | n-C13H27 |
| 37 (not part of invention) | Se | Se | n-C14H29 | n-C14H29 |
| 38 | S | Se | CH3 | CH3 |
| 39 | S | Se | C8H17 | C8H17 |
| 40 | S | Se | CH3 | C12H25 |
| 41 | Te | Te | CH3 | CH3 |
| 42 | Te | Te | C2H5 | C2H5 |
| 43 | Te | Te | n-C4H9 | n-C4H9 |
| 44 | Te | Te | t-C4H9 | t-C4H9 |
| 45 (not part of invention) | Te | Te | n-C6H13 | n-C6H13 |
| 46 (not part of invention) | Te | Te | n-C7H15 | n-C7H15 |
| 47 (not part of invention) | Te | Te | n-C5H17 | n-C8H17 |
| 48 (not part of invention) | Te | Te | n-C12H25 | n-C12H25 |
| 49 (not part of invention) | Te | Te | n-C14H29 | n-C14H29 |

| | | | | |
|---|---|---|---|---|
| * (not part of the invention) | | | | |

A field effect transistor (hereinafter, sometimes simply referred to as FET) of the present invention has two electrodes, i.e., a source electrode and a drain electrode, in contact with a semiconductor. The current flowing between the electrodes is controlled by the voltage applied to another electrode called a gate electrode.

Generally, in a field effect transistor, a structure in which a gate electrode is isolated by an insulating film, i.e., a metal-insulator-semiconductor (MIS) structure, is frequently used. A structure having a metal oxide film as the insulating film is called as a MOS structure. Others include a structure having a gate electrode via the Schottky barrier, i.e., an MES structure. In an FET using an organic semiconductor material, an MIS structure is frequently used.

Referring to the drawings, an organic field effect transistor according to the present invention will be more specifically described below. However, the present invention is not limited to these structures.
Fig. 1 shows schematic views illustrating field effect transistors (devices) according to several embodiments of the present invention. In each embodiment, reference number 1 represents a source electrode, 2 a semiconductor layer, 3 a drain electrode, 4 an insulating layer, 5 a gate electrode and 6 a substrate, respectively. It should be noted that, the arrangement of individual layers and electrodes can be appropriately modified depending upon a predetermined use of the device. A to D are each called a horizontal FET since current flows in parallel to a substrate. A is called a bottom-contact structure and B a top-contact structure. Furthermore, C shows a structure frequently used in formation of an organic single-crystal FET, in which source and drain electrodes and an insulating layer are formed on a semiconductor substrate and a gate electrode is further formed thereon. D shows a structure called a top & bottom-contact type transistor. E shows a schematic view of an FET having a vertical structure, i.e., a static induction transistor (SIT). In the SIT, since a current flow can spread in a two-dimensional way, a large amount of carriers can be transferred at a time. In addition, since a source electrode and a drain electrode are vertically arranged, the distance between them can be reduced and thereby high-speed response can be realized. Accordingly, the SIT can be preferably used for supplying a large amount of current or performing high-speed switching. No substrate is shown in E of Fig. 1. However, substrates are usually provided outside the source and drain electrodes respectively indicated by reference numbers 1 and 3 therein.

Structural elements in each embodiment will be described.
A substrate 6 has to keep carrying layers formed thereon without the detachment. For example, used be can an insulating material such as a resin board, a film, paper, glass, quartz and ceramic; a material in which an insulating layer is formed on a conductive substrate such as a metal and an alloy by a coating process or others; and a material consisting of a combination of a resin and an inorganic material. Examples of the resin film that can be used include polyethylene terephthalate, polyethylene naphthalate, polyether sulfone, polyamide, polyimide, polycarbonate, cellulose triacetate and polyether imide. If a resin film and paper is used, a device can have pliability and is improved in flexibility, lightness and usefulness. The thickness of a substrate is usually 1 µm to 10 mm and preferably 5 µm to 5 mm.

For the source electrode 1, drain electrode 3 and gate electrode 5, a conductive material is used. More specifically, used be can a metal such as platinum, gold, silver, aluminium, chromium, tungsten, tantalum, nickel, cobalt, copper, iron, lead, tin, titanium, indium, palladium, molybdenum, magnesium, calcium, barium, lithium, potassium and sodium, and an alloy containing them; a conductive oxide such as InO₂, ZnO₂, SnO₂ and ITO; a conductive polymer such as polyaniline, polypyrrole, polythiophene, polyacetylene, polyparaphenylene, vinylene and polydiacetylene; a semiconductor such as silicon, germanium and gallium arsenic; and a carbon material such as carbon black, fullerene, carbon nanotube and graphite. Furthermore, a conductive polymer and a semiconductor may have a dopant. Examples of the dopant include an inorganic acid such as hydrochloric acid and sulfuric acid; an organic acid having an acidic functional group such as sulfonic acid; a Lewis acid such as PF₅, AsF₅ and FeCl₃; a halogen atom such as iodine; and a metal atom such as lithium, sodium and potassium. Boron, phosphorus, arsenic, and the like are frequently used as a dopant for an inorganic semiconductor such as silicon. Furthermore, a composite conductive material wherein carbon black or metal particles are dispersed in the dopant, may be used.
Furthermore, the distance (channel length) between a source electrode and a drain electrode is an important factor for determining the characteristics of a device. The channel length is usually 0.1 to 300 µm and preferably 0.5 to 100 µm. If the channel length is short, the amount of current to be taken out increases but current leakage would be caused. Therefore, an appropriate channel length is required. The width (channel width) between a source electrode and a drain electrode is usually 10 to 10000 µm and preferably 100 to 5000 µm. The channel width can be further extended if a comb-like electrode structure is employed. The channel width may be appropriately designed depending upon a requisite current amount and a device structure.
The structure (shape) of each of a source electrode and a drain electrode will be described. The structures of the source and drain electrodes may be the same or different. When a bottom-contact structure is employed, it is generally preferred to form each electrode into a rectangular parallelepiped by a lithographical process. The length of an electrode may be the same as the channel width mentioned above. The width of the electrode is not particularly limited. However, in order to reduce the area of a device, the width is preferably as short as possible provided that the electrical properties can be stabilized. The width of an electrode is usually 0.1 to 1000 µm and preferably 0.5 to 100 µm. The thickness of an electrode is usually 0.1 to 1000 nm, preferably 1 to 500 nm, and more preferably 5 to 200 nm. To each of electrodes 1, 3, 5, wiring is connected. The wiring is made of the same as or a similar material to that as used in the electrodes.

For an insulating layer 4, an insulating material is used. Specifically, used be can a polymer such as polyparaxylylene, polyacrylate, polymethyl methacrylate, polystyrene, polyvinyl phenol, polyamide, polyimide, polycarbonate, polyester, polyvinyl alcohol, polyvinyl acetate, polyurethane, polysulfone, an epoxy resin and a phenol resin and a copolymer thereof; a metal oxide such as silicon dioxide, aluminum oxide, titanium oxide and tantalum oxide; a ferroelectric metal oxide such as SrTiO₃ and BaTiO₃; a nitride such as silicon nitride and aluminum nitride; a sulfide; a dielectric substance such as fluoride; or a polymer in which particles of the dielectric substance are dispersed. The film thickness of a insulating layer 4 varies depending upon the materials. However, it is usually 0.1 nm to 100 µm, preferably 0.5 nm to 50 µm, and more preferably 1 nm to 10 µm.

For a material for a semiconductor layer 2, a compound represented by Formula (1) is used. A mixture of the compounds may be used. However, in a semiconductor layer, a compound(s) represented by Formula (1) is contained usually in an amount of 50 wt% or more, preferably 80 wt% or more, and further preferably 95 wt% or more.
In order to improve the characteristics of a field effect transistor or impart other characteristics, if necessary, another type of organic semiconductor material and various additives may be added. Furthermore, the semiconductor layer 2 may have a multilayer structure.
In a field effect transistor of the present invention, at least one compound represented by Formula (1) is used as a semiconductor material, substantially only a compound(s) represented by Formula (1) is preferably used as a semiconductor material, and only a single compound represented by the above Formula (1) is particularly preferably used as a semiconductor material, which is better than a mixture of two or more of the compounds. However, in order to improve the characteristics of a transistor, as mentioned above, additives such as a dopant may be added.
Such additives may be added usually within a range of 0.01 to 10 wt%, preferably 0.05 to 5 wt%, and more preferably 0.1 to 3 wt% if the total amount of semiconductor materials was regarded as 1.
Furthermore, a semiconductor layer may also have a multilayer structure but more preferably a single layer structure.
The thinner the film of the semiconductor layer 2, the more preferable as long as a necessary function is not impaired. This is because in horizontal field effect transistors as shown in A, B and D, the characteristics do not depend upon the thickness as long as it is above a certain level; whereas leaked current increases in many cases, as the thickness increases. In order to exert necessary functions, the thickness of a semiconductor layer is usually 1 nm to 10 µm, preferably 5 nm to 5 µm, and more preferably 10 nm to 3 µm.

In a field effect transistor of the present invention, if necessary, another layer may be provided, for example, between a substrate layer and a insulating film layer, between a insulating film layer and a semiconductor layer, or on the outer surface of the device. For instance, when a protective layer is formed directly or via another layer on a semiconductor layer, an atmospheric influence caused by humidity or the like can be reduced. Further, the electrical properties can be advantageously stabilized, and for instance the ON/OFF ratio of a device can be increased.
The materials for the protective layer, not being limited, preferably include a film made of a resin such as an epoxy resin, an acryl resin, e.g., polymethyl methacrylate, polyurethane, polyimide, polyvinyl alcohol, a fluorine resin and a polyolefin; a film of an inorganic oxide such as silicon oxide, aluminum oxide and silicon oxide; and a dielectric film such as nitride film. In particular, a resin (polymer) with low permeability of oxygen or water or small water absorption is preferred. A protective material recently developed for an organic EL display can be also used. The thickness of a protective layer can be optionally selected depending upon the intended use, and is usually 100 nm to 1 mm.

Furthermore, the characteristics of a device can be improved by previously applying a surface treatment onto a substrate, on which a semiconductor layer is to be layered, or an insulating layer. For example, the characteristics of a film to be formed on a substrate can be improved by modifying, for example, the hydrophilicity/hydrophobicity of the substrate surface. In particular, the characteristics of an organic semiconductor material may sometimes greatly vary depending upon the conditions of a film such as molecular orientation. A surface treatment of a substrate or the like can modify molecular orientation of the interface portion between a substrate or the like and a semiconductor layer to be formed thereon and reduce the number of trap sites on the substrate or a insulating layer, whereby, characteristics such as carrier mobility would be improved.
The trap site refers to a functional group such as a hydroxy group on an untreated substrate. When such a functional group exists there, electrons are attracted to the functional group, whereby carrier mobility decreases. Accordingly, it is most often effective to reduce the number of trap sites in improving the characteristics such as carrier mobility.
Examples of such a substrate treatment include a hydrophobing treatment with hexamethyldisilazane, cyclohexene, octyltrichlorosilane, octadecyltrichlorosilane or the like; an acid treatment with hydrochloric acid, sulfuric acid, acetic acid or the like; an alkaline treatment with sodium hydroxide, potassium hydroxide, calcium hydroxide, ammonia or the like; an ozone treatment; a fluorination treatment; a plasma treatment with oxygen, argon or the like; the formation of a Langmuir-Blodgett film; the formation of a thin film of another insulator or a semiconductor; a mechanical treatment; an electrical treatment such as corona discharge; and a rubbing treatment with a fiber.

In these embodiments, appropriate examples of a method for providing layers such as a substrate layer and an insulating film layer, or an insulating film layer and a semiconductor layer include a vacuum evaporation method, a sputtering method, a coating method, a printing method and a sol-gel method.

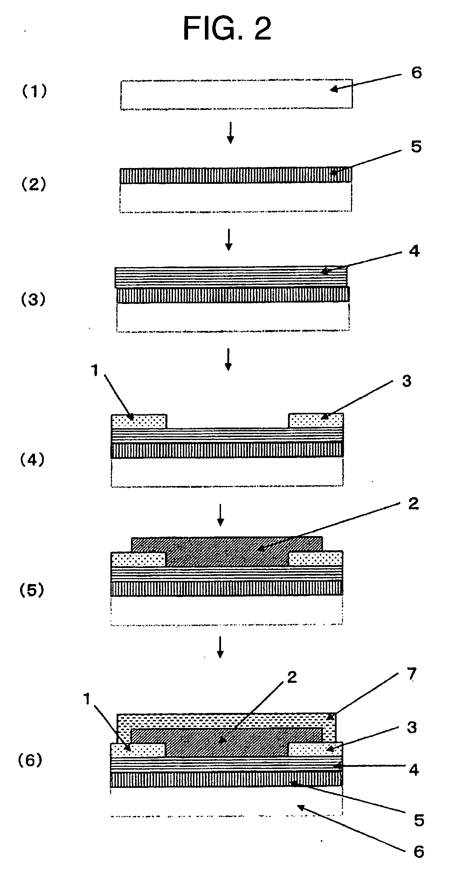

Next, a method for manufacturing a field effect transistor according to the present invention will be described below by way of a bottom-contact type field effect transistor (FET) shown in embodiment A of Fig. 1 and with reference to Fig. 2.
The manufacturing method can be similarly applied also to field effect transistors according to other embodiments mentioned above.

### Substrate and substrate treatment

A field effect transistor of the present invention is manufactured by providing various requisite layers and electrodes on a substrate 6 (see Fig. 2 (1)). The substrate is as described above. To the substrate, the aforementioned surface treatments can be also applied. The substrate 6 is preferably as thin as possible provided that necessary function is not affected. The thickness varies depending upon the material but is generally 1 µm to 10 mm and preferably 5 µm to 5 mm. Furthermore, if necessary, the substrate may also serve as an electrode.

### Formation of gate electrode

A gate electrode 5 is formed on the substrate 6 (see Fig. 2 (2)). The materials for the electrode are as described above. In forming an electrode film, various methods can be used. Examples thereof include a vacuum evaporation method, a sputtering method, a coating method, a heat-transfer method, a printing method and a sol-gel method. Patterning may be preferably conducted, as needed, during or after a film formation process so as to make a desired shape. Various types of patterning methods may be used. For example, a photolithography conducted by a combination of patterning and etching of a photoresist can be employed. Alternatively, patterning can be conducted also by use of a printing method such as inkjet printing, screen printing, off-set printing and relief printing; a soft lithographic method such as micro-contact printing; and a combination method thereof. The thickness of the gate electrode 5 varies depending upon the material. However, it is usually 0.1 nm to 10 µm, preferably 0.5 nm to 5 µm, and more preferably 1 nm to 3 µm. Furthermore, when a gate electrode also serves as a substrate, the thickness may be larger than the aforementioned film thickness.

### Formation of insulating layer

An insulating layer 4 is formed on the gate electrode 5 (see Fig. 2 (3)). The materials are as described above. The insulating layer 4 can be formed by various methods. Examples thereof include a coating method such as spin coating, spray coating, dip coating, cast coating, bar coating and blade coating; a printing method such as screen printing, off-set printing and inkjet; and a dry-process method such as a vacuum evaporation method, a molecular beam epitaxial growth method, an ion cluster beam method, an ion plating method, a sputtering method, an atmospheric plasma method and a CVD method. Other examples include a sol-gel method and a method of forming an oxide film on a metal material, e.g., alumite on aluminum or a silicon dioxide on silicon.
At the portion at which an insulating layer and a semiconductor layer are in contact, a predetermined surface treatment can be applied to the insulating layer in order to properly orient molecules constituting a semiconductor, e.g., molecules of a compound represented by Formula (1) as mentioned above, on the interface between the two layers. A method for the surface treatment may be the same as that to be applied to a substrate. The insulating layer 4 is preferably as thin as possible provided that the function is not impaired. The thickness is usually 0.1 nm to 100 µm, preferably 0.5 nm to 50 µm, and more preferably 5 nm to 10 µm.

### Formation of source electrode and drain electrode

A source electrode 1 and drain electrode 3 can be formed in the same manner as used for forming the gate electrode 5 (see Fig. 2 (4)).

### Formation of semiconductor layer

As described above, an organic material containing one of the compounds represented by Formula (1) or a mixture of two or more of them usually in a total amount of 50 wt% or more is used as a semiconductor one. A semiconductor layer can be formed by various methods, which are roughly classified into a forming method by a vacuum process such as a sputtering method, a CVD method, a molecular beam epitaxial growth method and a vacuum evaporation method; and a forming method by a solution process such as a coating method, e.g., a dip coat method, a die coater method, a roll coater method, a bar coater and a spin coat method, an inkjet method, a screen printing method, an off-set printing method and a micro-contact printing method.

When a semiconductor layer is formed by using a compound represented by Formula (1) as a semiconductor material, it is more preferred to form an organic semiconductor layer by a vacuum process rather than by a solution process, and a vacuum evaporation method is further preferable.

A method for obtaining an organic semiconductor layer by forming an organic material into a film by a vacuum process will be described.
A method as follows, namely, a vacuum evaporation method, is preferably employed: an organic material as described above is heated under vacuum in a crucible or a metal boat, and the vaporized organic material is allowed to adhere (deposit) onto a substrate (exposed portions of an insulating layer, a source electrode and a drain electrode). In this method, the degree of vacuum is usually 1.0 x 10⁻¹ Pa or less and preferably 1.0 x 10⁻³ Pa or less. Furthermore, the characteristics of a organic semiconductor film and accordingly the ones of a field effect transistor therefrom may vary depending upon the substrate temperature during the evaporation process. Therefore, it is preferred that the substrate temperature is carefully selected. The substrate temperature during a evaporation process is usually 0 to 200°C, preferably 10 to 150°C, more preferably 15 to 120°C, further preferably 25 to 100°C, and particularly preferably 40 to 80°C. Furthermore, in the case of a compound represented by Formula (1) wherein X¹ and X² are the same and represent a sulfur atom or selenium atom, and R¹ and R² are the same and represent a methyl group, a semiconductor film is preferably formed by deposition or the like at a substrate temperature of 40 to 80°C, and particularly preferably at a substrate temperature of 50 to 80°C.
Furthermore, the deposition rate is usually 0.001 nm/second to 10 nm/second, and preferably 0.01 nm/second to 1 nm/second. The thickness of an organic semiconductor layer made of an organic material is usually 1 nm to 10 µm, preferably 5 nm to 5 µm, and more preferably 10 nm to 3 µm.
Note that a evaporation method of heating and evaporating an organic material for forming a semiconductor layer and depositing it onto a substrate, may be replaced with a sputtering method of bombarding a target material with an accelerated ion such as argon to eject atoms of the material, thereby depositing the atoms onto the substrate.

The semiconductor material of the present invention is from an organic compound with a relatively low molecular weight. Therefore, such a vacuum process may be preferably used. Such a vacuum process requires slightly expensive equipment but has advantages in that a uniform film can be easily obtained with good film-formability.

Ink for use in manufacturing a semiconductor device can be prepared as mentioned above and is applied onto a substrate (exposed portions of an insulating layer, a source electrode and a drain electrode). A method of applying ink includes a coating method such as casting, spin coating, dip coating, blade coating, wire bar coating and spray coating; a printing method such as inkjet printing, screen printing, off-set printing and relief printing; a soft lithographic method such as a micro-contact printing method; and a combination thereof.
Furthermore, a similar method to the method of applying ink may be employed and includes a Langmuir project method, in which a monomolecular film of a semiconductor layer is produced by dropping the ink as mentioned above onto a water surface and is then transferred to and put on a substrate, and a method of sandwiching a liquid crystal or a melt-state material between two substrates or introducing it into the space between two substrates by a capillary phenomenon.
An organic semiconductor layer prepared by this method is preferably as thin as possible provided that a function is not impaired. As the thickness gets increased, leaked current may increase. The thickness of an organic semiconductor layer formed by this method is usually 1 nm to 10 µm, preferably 5 nm to 5 µm, and more preferably 10 nm to 3 µm.

The formed semiconductor layer (see Fig. 2 (5)) can be further improved in the characteristics by a post treatment. For instance, the characteristics would be improved and stabilized as follows: a heat treatment may relax strain produced in a film formation process, reduce the number of pin holes, and control alignment/orientation of molecules in a film. Such a heat treatment would effective for manufacturing a field effect transistor of the present invention in terms of improving the characteristics. The heat treatment may be conducted by heating a substrate after a semiconductor layer is formed. The treatment temperature is not particularly limited but is usually from room temperature to about 150°C, preferably 40 to 120°C, and further preferably 45 to 100°C. The treatment time is not particularly limited but is usually 1 minute to 24 hours, and preferably 2 minutes to about 3 hours. The treatment atmosphere may be air or an inert atmosphere such as nitrogen, argon or the like.
Furthermore, other post treatment methods may be employed and include a treatment with an oxidative or reducible gas such as oxygen or hydrogen, or with an oxidative or reducible liquid, which can induce a change in the characteristics. In many cases, these methods are used for increasing or decreasing the carrier density of a film, or others.

Furthermore, the characteristics of a semiconductor layer can be changed by adding a small amount of element, atomic group, molecule and polymer to the semiconductor layer in accordance with a socalled doping methodology. For example, an acid such as oxygen, hydrogen, hydrochloric acid, sulfuric acid and sulfonic acid; a Lewis acid such as PF₅, AsF₅ and FeCl₃; a halogen atom such as iodine; a metal atom such as sodium and potassium can be employed for doping. The doping can be conducted by bringing a gas of the aforementioned substance into contact with a semiconductor layer, immersing a semiconductor layer in a solution, or subjecting a semiconductor layer to an electrochemical doping process. These dopings do not have to be conducted after a semiconductor layer is formed, but a dopant may be added during the synthesis of a semiconductor material. In the case where a semiconductor layer is formed by ink for use in manufacturing a semiconductor device, a dopant can be added to the ink. Further, a dopant can be added, for example, in a step of forming a precursor thin film as disclosed in Patent Document 2. Furthermore, a material for dioping may be added to a material for forming a semiconductor layer in a evaporation process in order simultaneously evaporate them, or a material for doping may be mixed with an ambient atmosphere used in forming a semiconductor layer: a semiconductor layer is formed under an environment containing a doping material. Furthermore, the doping can be conducted by accelerating ions in vacuum and bombarding them to a film.

These doping methods would result in a change of electrical conductivity due to an increased or decreased carrier density, a change of carrier polarity (p-type or n-type), a change of Fermi level and the like. Such doping is frequently used in a semiconductor device derived from an inorganic material, particularly, silicon or the like.

### Protective layer

The formation of a protective layer 7 on an organic semiconductor layer has advantages in that influence of the outer air can be minimized and the electrical characteristics of an organic field effect transistor can be stabilized (see Fig. 2 (6)). Materials for the protective layer 7 are as mentioned above.
The protective layer 7 may have an appropriate thickness depending upon a intended use but usually 100 nm to 1 mm.
Various methods may be employed for forming the protective layer. When the protective layer is made of a resin, such methods include a method of applying a resin solution and then drying it to form a resin film, and a method of applying or evaporating a resin monomer and then polymerizing the monomer. After the formation of the film, it may be crosslinking-treated. When a protective layer is made of an inorganic substance, the following methods can be also used: a forming method by a vacuum process such as a sputtering method and a evaporation method, and a forming method by a solution process such as a sol-gel method.
In a field effect transistor of the present invention, a protective layer can be provided not only on an organic semiconductor layer but also between layers, if necessary. These protecting layers may serve to stabilize the electrical characteristics of the organic field effect transistor.

According to the present invention, an organic material is used as a semiconductor material, whereby a relatively low-temperature process can be employed. Therefore, a flexible material such as a plastic plate and a plastic film, which cannot be used in the conditions exposed to high temperature, can be also used as a substrate. As a result, an irrefrangible device excellent in lightness and flexibility can be manufactured and used as a switching element of an active matrix in a display, and others. The display includes a liquid crystal display, a polymer dispersed liquid crystal display, an electrophoretic display, an EL display, an electrochromic display and a particle rotation display.

A field effect transistor of the present invention can be also used as a digital device or an analog device such as a memory circuit device, a signal driver circuit device and a signal processing circuit device. Furthermore, these can be used in combination to manufacture an IC card and an IC tag. Furthermore, a field effect transistor of the present invention can be changed in the characteristics by an external stimulus such as a chemical substance and thus can be also used as an FET sensor.

The operating characteristics of a field effect transistor can be determined, for example, based on the carrier mobility and conductivity of its semiconductor layer, the capacitance of its insulating layer and the structures of the device, e.g., the distance between its source and drain electrodes and the widths thereof, the thickness of the insulating layer, and others. A semiconductor material for a field effect transistor is more preferable, the higher the carrier mobility of the resulting semiconductor layer. A compound represented by Formula (1) has good film formability. Furthermore, a pentacene derivative is labile in the air and difficult to handle, i.e., decomposed with moisture in the air. However, when a compound represented by Formula (1) of the present invention is used as a semiconductor material, it has an advantage in that the resulting semiconductor layer also has high stability and a long life. Furthermore, a transistor having a semiconductor layer made of a compound represented by Formula (1) has a low threshold voltage. Therefore, when such a transistor is used in practice, a driving voltage is reduced and power consumption is reduced thereby saving more energy compared to conventional ones. As a result, the transistor can be effectively used, for example, in a mobile display with a rechargeable battery, of which longer operation time is required. In addition, a reduced threshold voltage reduces energy consumption and further lowers a barrier against an electric charge which is transferred from an electrode to a semiconductor film. Thus, it is expected to bring out increased durability of a semiconductor element and a semiconductor device having it.

### EXAMPLES

The present invention will be more specifically described by way of examples below. However, the present invention should not be limited to them. In the examples, unless otherwise specified, the term "parts" represents "parts by mass", "%" represents " "% by mass", and "Compound No." represents "Compound No." in the Table 1 above.
The reaction temperature, unless otherwise specified, refers to the inner temperature of a reaction system.
Compounds obtained in Synthesis Examples were subjected, as appropriate, to MS (mass spectrometry) and measurements for maximum absorption (λ max) and mp (melting point) to determine their structural formulas. Measurement apparatuses are as follows.
MS spectrum: Shimadzu QP-5050A
Absorption spectrum: Shimadzu UV-3150

### Synthesis Example 1

### Synthesis of 2,6-bis(p-methylphenyl)benzo[1,2-b:4,5-b']dithiophene (Compound No. 1, not part of the invention)

1,4-Dibromo-2,5-bis(p-methylphenylethynyl)benzene (39.9 g, 86 mmol) was dissolved in N,N-dimethylacetamide (150 ml), and a 70% aqueous solution of NaSH·nH₂O (20.7 g, 527 mmol) was added and stirred at 140 to 150°C for 6 hours. The reaction mixture was cooled to room temperature and methanol (300 ml) was added. A precipitated solid substance was taken out by filtration and washed with methanol (300 ml), water (300 ml) and methanol (300 ml) sequentially in this order. The resultant solid substance was dried and then purified by sublimation to obtain Compound No. 1(23.0 g, yield 72.1%).
MS (70 eV, EI) m/z=370(M+)
Maximum absorption: 414 nm (vapor deposition film)

### Synthesis Example 2

### Synthesis of 2,6-bis(p-ethylphenyl)benzo[1,2-b:4,5-b']dithiophene (Compound No. 2)

The same procedure was repeated except that 1,4-dibromo-2,5-bis(p-ethylphenylethynyl)benzene (39.9 g, 86 mmol) in Synthesis Example 1 was replaced with 1,4-dibromo-2,5-bis(p-ethylphenylethynyl)benzene (43.3 g, 86 mmol) to obtain Compound No. 2 (26.1 g, yield 76.2%).
MS (70 eV, EI) m/z=398(M+)
Maximum absorption: 416 nm (vapor deposition film)

### Synthesis Example 3

### Synthesis of 2,6-bis(p-(n-octyl)phenyl)benzo[1,2-b:4,5-b']dithiophene (Compound No. 11 , not part of the invention)

The same procedure was repeated except that 1,4-dibromo-2,5-bis(p-ethylphenylethynyl)benzene (39.9 g, 86 mmol) in Synthesis Example 1 was replaced with 1,4-dibromo-2,5-bis(p-(n-octyl)phenylethynyl)benzene (3.96 g, 6.0 mmol) to obtain Compound No. 11 (2.64 g, yield 77.6%).
MS (70 eV, EI) m/z=566(M+)

### Synthesis Example 4

### Synthesis of 2,6-bis(p-methylphenyl)benzo[1,2-b:4,5-b']diselenophene (Compound No. 21)

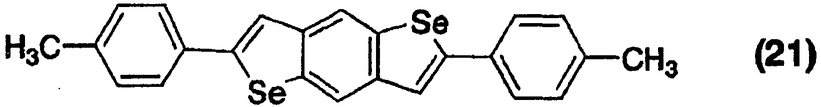

In a nitrogen atmosphere, under cooling with ice water, a selenium powder (118.5 mg, 1.5 mmol) and NaBH₄ (56.7 mg, 1.5 mmol) were dissolved in ethanol (5 mL) and stirred for 30 minutes. 1,4-Dibromo-2,5-bis(p-methylphenylethynyl)benzene (232 mg, 0.5 mmol) and anhydrous N-methylpyrrolidone (12 mL) were added, and the solution was elevated to room temperature and stirred at 190°C for 22 hours. The reaction solution was poured in a saturated aqueous ammonium chloride solution (100 mL). A precipitated solid substance was taken out by filtration and washed with water (200 mL), acetone (200 mL) and methylene chloride (200 mL) sequentially in this order. The resultant solid substance was dried and purified by sublimation to obtain Compound No. 21 (109.5 mg, 47%) as a yellow solid substance.
MS (70 eV, EI) m/z=464(M+)
Maximum absorption: 432 nm (vapor deposition film)

### Synthesis Example 5

### Synthesis of 2,6-diphenylbenzo[1,2-b:4,5-b']dithiophene (Comparative Compound No. 100)

To NMP (N-methyl-2-pyrrolidone, 30 ml) and 1,4-dibromo-2,5-bisphenyl benzene (6.54 g, 15 mmol), 70% NaSH·nH₂O (3.60 g, 92 mmol) and copper iodide (0.14 g, 0.8 mmol) were added and reacted at an inner temperature of 140 to 150°C for 3 hours. The solution was allowed to cool and toluene (100 ml) was added. A precipitated solid substance was taken out by filtration and washed with toluene (30 ml) and methanol (30 ml) and dried. The resultant compound was purified by sublimation to obtain Comparative Compound No. 100 (4.37 g, yield 85%) shown below.
MS (70 eV, EI) m/z=342(M+)

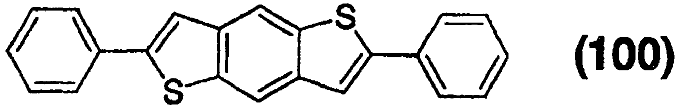

### Synthesis Example 6

### Synthesis of 2,6-diphenylbenzo[1,2-b:4,5-b']diselenophene (Comparative Compound No. 101)

In a nitrogen atmosphere, selenium powder (98.7 mg, 1.25 mmol) and NaBH₄ (47.3 mg, 1.25 mmol) were dissolved in ethanol (5 ml) and stirred at 5°C for 30 minutes. To the solution, 1,4-dibromo-2,5-bisphenyl benzene (216 mg, 0.50 mmol) and NMP (12 ml) were added and the mixture was heated at an inner temperature of 190°C for 20 hours. The reaction solution was poured in water. A precipitated solid substance was taken out by filtration and then washed with methanol (30 ml) and acetone (30 ml). After drying it, the resultant compound was purified by sublimation to obtain a yellow crystal compound 101 (114 mg, yield 52%).
MS (70 eV, EI) m/z=436(M+)

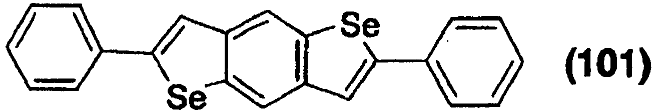

### Example 1

An n-doped silicon wafer (surface resistance 0.02 Ω·cm or less) with a 300 nm-SiO₂ thermal oxidation film obtained by the treatment with hexamethylene disilazane was placed in a vacuum evaporation apparatus and the apparatus was evacuated until a vacuum of 5.0 x 10⁻³ Pa or less. To the electrode, Compound No. 1 was evaporated up to a thickness of 50 nm by a resistive heating evaporation method at a substrate temperature of about 25°C to form a semiconductor layer (2). Subsequently, a shadow mask for forming an electrode was attached to the substrate and the substrate was placed in the vacuum evaporation apparatus. The apparatus was evacuated until a vacuum of 1.0 x 10⁻⁴ Pa or less and gold electrodes, i.e., a source electrode (1) and a drain electrode (3) was formed with a thickness of 40 nm by a resistive heating evaporation method to obtain a TC (top-contact) type of field effect transistor according to the present invention.

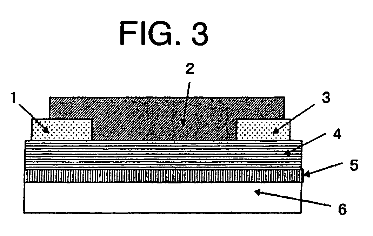

Note that, in the field effect transistor in this example, the thermal oxidation film of the n-doped silicon wafer serves as an insulating layer (4) and the n-doped silicon wafer serves as not only a substrate (6) but also a gate electrode (5) (see Fig. 3).
The obtained field effect transistor was placed in a prober and the semiconductor characteristics were measured by a semiconductor parameter analyzer 4155C (manufactured by Agilent). As the semiconductor characteristics, a drain currentdrain voltage was measured by scanning a gate voltage from 10V to -100V at the intervals of 20V, and scanning a drain voltage from 10V to -100V. As a result, current saturation was observed. From the obtained voltage-current curve, it was found that the device is a p-type semiconductor having a carrier mobility of 0.31 cm²/Vs and a threshold voltage of -20.7 V.

### Example 2

A TC type of field effect transistor was obtained in the same manner as in Example 1 except that Compound No. 1 used in Example 1 was replaced with Compound No. 21 obtained in Synthesis Example 4.

### Example 3

On an n-doped silicon wafer (surface resistance 0.02 Ω·cm or less) with a 300 nm of SiO₂ thermal oxidation film obtained by the treatment with hexamethylene disilazane, a resist material was applied and exposed to light to make patterning. Then, chromium (1 nm) and further gold (40 nm) were deposited there. Subsequently, the resist was removed to form a source electrode (1) and a drain electrode (3) (a comb-like electrode having a channel length: 25 µm x channel width: 2 mm x 20 pieces). A silicon wafer having the electrode was placed in a vacuum evaporation apparatus and the apparatus was evacuated until a vacuum of 5.0 x 10⁻³ Pa or less. Compound No. 1 (50 nm in thickness) was evaporated on the electrodes by a resistive heating evaporation method at a substrate temperature of about 25°C to form the semiconductor layer (2). Consequently, a BC (bottom-contact) type of field effect transistor according to the present invention was obtained. In the field effect transistor in this Example, the thermal oxidation film of the n-doped silicon wafer serves as a insulating layer (4) and the n-doped silicon wafer serves as not only a substrate (6) but also a gate layer (5) (see Fig. 3). The semiconductor characteristics were measured in the same manner as in Example 1, current saturation was observed. From the obtained voltage-current curve, it was found that the device is of a p-type semiconductor having a carrier mobility of 0.28 cm²/Vs and a threshold voltage of -29.7 V.

### Example 4

A BC-type of field effect transistor was obtained in the same manner as in Example 3 except that Compound No. 1 used in Example 3 was replaced with Compound No. 2 obtained in Synthesis Example 2.

### Example 5

A BC-type of field effect transistor was obtained in the same manner as in Example 3 except that Compound No. 1 used in Example 3 was replaced with Compound No. 11 obtained in Synthesis Example 3.

### Example 6

A BC-type of field effect transistor was obtained in the same manner as in Example 3 except that the substrate temperature in the evaporation of Compound No. 1 in Example 3 was changed to 60°C.

### Example 7

A BC-type of field effect transistor was obtained in the same manner as in Example 3 except that Compound No. 1 in Example 3 was replaced with Compound No. 21 obtained in Synthesis Example 4 and the substrate temperature in the evaporation was changed to 60°C.

### Comparative Example 1

A TC type of field effect transistor was obtained in the same manner as in Example 1 except that Compound No. 1 in Example 1 was replaced with Compound No. 100 obtained in Synthesis Example 5 (a compound represented by Formula (1) wherein R¹ and R² are hydrogen atoms, both X¹ and X² are sulfur atoms).

### Comparative Example 2

A TC type of field effect transistor was obtained in the same manner as in Example 1 except that Compound No. 1 in Example 1 was replaced with Compound No. 101 obtained in Synthesis Example 6 (a compound represented by Formula (1) wherein R¹ and R² are hydrogen atoms, both X¹ and X² are selenium atoms).

### Comparative Example 3

A BC type of field effect transistor was obtained in the same manner as Example 3 except that Compound No. 1 in Example 3 was replaced with Compound No. 101.

### Comparative Example 4

A BC type of field effect transistor was obtained in the same manner as in Example 3 except that Compound No. 1 in Example 3 was replaced with Compound No. 101 and the substrate temperature in the evaporation of the compound was changed to 60°C.

The field effect transistors obtained in Examples 2 to 7 and Comparative Examples 1 to 4 were measured for the semiconductor characteristics in the same manner as in Example 1. In all the transistors, current saturation was observed. The obtained voltage-current curve demonstrated that all the devices are of a p-type semiconductor. The structure, substrate temperature, carrier mobility and threshold voltage of the obtained devices are collectively shown in Table 2 to Table 4.

**[Table 2]**

| Table 2 | Compound No. | Device Structure | Substrate temperature (°C) | Mobility (cm²/Vs) | Threshold voltage (V) |
|---|---|---|---|---|---|
| Example 1 | 1 | TC | 25 | 0.31 | -20.7 |
| Example 2 | 21 | TC | 25 | 0.34 | -21.4 |
| Comparative Example 1 | 100 | TC | 25 | 0.21 | -45.1 |
| Comparative Example 2 | 101 | TC | 25 | 0.10 | -36.6 |

**[Table 3]**

| Table 3 | Compound No. | Device Structure | Substrate temperature (°C) | Mobility (cm²/Vs) | Threshold voltage (V) |
|---|---|---|---|---|---|
| Example 3 | 1 | BC | 25 | 0.28 | -29.7 |
| Example 4 | 2 | BC | 25 | 0.05 | -30.9 |
| Example 5 | 11 | BC | 25 | 0.05 | -42.9 |
| Comparative Example 3 | 100 | BC | 25 | 0.05 | -49.0 |

**[Table 4]**

| Table 4 | Compound No. | Device Structure | Substrate temperature | Mobility | Threshold voltage |
|---|---|---|---|---|---|
| Example 6 | 1 | BC | 60 | 0.49 | -14.6 |
| Example 7 | 21 | BC | 60 | 0.56 | -18.1 |
| Comparative Example 4 | 101 | BC | 60 | 0.43 | -29.3 |

The results of Table 2 to Table 4 will be described below in order.

Example 1 and Comparative Example 1 in Table 2 relate to use of compounds represented by Formula (1) wherein X¹ and X² are sulfur atoms, and R¹ and R² is methyl in the former but a hydrogen atom in the latter, respectively. Furthermore, Example 2 and Comparative Example 2 relate to use of compounds wherein X¹ and X² are selenium atoms, and R¹ and R² is methyl in the former but a hydrogen atom in the latter, respectively. Both of these devices are of top-contact type transistors.
As is apparent from Table 2, the device of Example 1 had about 48% higher carrier mobility and an about 54% lower threshold voltage than that of Comparative Example 1. It is therefore apparent that the transistor of the present invention is extremely excellent in view of practical use such as power consumption. Furthermore, the results of Example 2 and Comparative Example 2 are similar to them. It was confirmed that the device of Example 2 had a 240% higher carrier mobility and an about 42% lower threshold voltage.
Furthermore, Examples 1 and 2, which relate to top-contact type transistors from the compounds having the same structure except that a sulfur atom is contained in the former whereas a selenium atom in the latter, demonstrated that both the devices had almost the same characteristics.

Table 3 shows the experimental results of the characteristics of the BC type transistors. Examples 3 to 5 relates to compounds represented by Formula (1) wherein X¹ and X² are sulfur atoms; and R¹ and R² are methyl, ethyl, and n-octyl, respectively. Comparative Example 3 relates to the same compound as in Comparative Example 1, i.e., a compound represented by Formula (1) wherein X¹ and X² are sulfur atoms and R¹ and R² are hydrogen atoms.
The device of Example 3 exerted 5.6-times greater carrier mobility than that of Comparative Example 3, but those of Examples 4 and 5 did the same as that of Comparative Example 3.
However, significant differences between them were found in terms of threshold voltage. It was confirmed that the threshold voltages thereof are reduced by at least about 12% and by up to about 39% relative to that of comparative Example 3.
Furthermore, other compounds represented by Formula (1) but different in the chain length of R¹ and R² alkyls brought different characteristics into the resulting transistors. More specifically, it was found that the shorter the alkyl chain of a compound, the lower the threshold voltage. However, they exerted an about 13% lower threshold voltage than the transistor of Comparative Example 3 using a compound represented by Formula (1) in which R¹ and R² are hydrogen atoms.
Furthermore, the transistor of Example 3 using a methyl derivative and the transistor of Example 4 using an ethyl derivative exerted a similar threshold voltage but differed in the carrier mobility by 5.6 times. It was found that a methyl derivative is the most excellent.

Example 3 and Example 6 respectively shown in Table 3 and Table 4 relate to the BC type transistors using the same compound but different in the substrate temperatures. The former was formed at 25°C and the latter at 60°C.
The transistor of Example 6 exerted about 1.8 times-greater carrier mobility and an about 51% lower threshold voltage than that of Example 3. These results at least demonstrated that when a semiconductor layer is made of a methyl derivative, the substrate temperature of 60°C has an advantage. Thus, it is important to carefully select the substrate temperature in forming a semiconductor layer.
Example 7 in Table 4 relates to a BC type of transistor manufactured by using a compound represented by Formula (1) wherein X¹ and X² are selenium atoms and X¹ and X² are methyl groups, and forming a semiconductor layer at a substrate temperature of 60°C, whereas Comparative Example 4 relates to a BC type of transistor manufactured by using a compound wherein X¹ and X² are selenium atoms and R¹ and R² are hydrogen atoms, and forming a semiconductor layer at a substrate temperature of 60°C. The comparison thereof demonstrated that even if a semiconductor layer is formed at a substrate temperature of 60°C, a methyl derivative greatly increases carrier mobility and reduces threshold voltage than the other. Furthermore, it was found that a semiconductor layer which is formed on a BC type of substrate by using a compound represented by Formula (1) wherein X¹ and X² are selenium atoms also brings excellent characteristics into the transistor.

### Example 9 (Wet heat resistance test)

BC type of field effect transistors obtained in Example 3 and Comparative Example 3, using compound 1 and compound 100, were separately placed in wet heat testers having a humidity of 80% and a temperature of 40°C. Carrier mobility was measured at the initial time, one hour later, and 24 hours later. The results are shown in Table 5. Relative mobility values are shown based on the carrier mobility (100%) at the initial time.

**[Table 5]**

| Table 5 | Compound | Initial time | One hour later | 24 hours later |
|---|---|---|---|---|
| Example 3 | 1 | 100 | 48 | 13 |
| Comparative Example 3 | 100 | 100 | 10 | 1 |

As is apparent from the results, the transistor using compound 100 with no alkyl group only exerted, one hour late, 10% of the carrier mobility at the initial time. In contrast, the transistor using compound 1 with a methyl group exerted, even 24 hours later, 13% of the carrier mobility at the initial time which demonstrated high durability. Thus, it was demonstrated that a methyl derivative imparts excellent durability under wet heat conditions.

### Example 10 (Heat resistance test)

BC type field effect transistors obtained in Example 3 and Comparative Example 3, using compound 1 and compound 100, were separately placed in heat resistance testers having a temperature of 120°C. Carrier mobility was measured at the initial time, one hour later, and 24 hours later. The results are shown in Table 6. Relative mobility values are shown based on the carrier mobility (100%) at the initial time.

**[Table 6]**

| table 6 | Compound | Initial time | One hour later | 24 hours later |
|---|---|---|---|---|
| Example 3 | 1 | 100 | 84 | 36 |
| Comparative Example 3 | 100 | 100 | 28 | 19 |

As is apparent from the results, the transistor using compound 100 with no alkyl group only exerted, one hour later, 28% of the carrier mobility at the initial time. In contrast, the transistor using compound 1 with a methyl group exerted 84% thereof. 24 hours later, the two transistors exerted 19% and 36% of the carrier mobility values at the initial time, respectively. Therefore, it was demonstrated that a methyl derivative brings out excellent durability under high temperature conditions.

As mentioned above, it was elucidated that such derivatives having an alkyl group have high durability under high temperature conditions and wet heat conditions.

## Claims

1. A field effect transistor **characterized by** having a semiconductor layer formed from at least one compound represented by the following Formula (1): 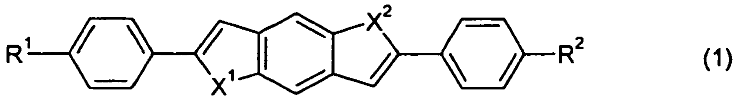 wherein, X¹ and X² each independently represent a sulfur atom, a selenium atom or a tellurium atom and R¹ and R² each independently represent a saturated or unsaturated linear, branched or cyclic C1-C14 alkyl group, with the proviso that when both of X¹ and X² are a selenium atom, R¹ and R² each independently represent a saturated or unsaturated linear, branched or cyclic C1-C4 alkyl group.

2. The field effect transistor according to claim 1, **characterized in that** R¹ and R² of Formula (1) are each independently a saturated or unsaturated linear, branched or cyclic C1-C8 alkyl group, with the proviso that, when both of X¹ and X² are a selenium atom, R¹ and R² each independently represent a saturated or unsaturated linear, branched or cyclic C1-C4 alkyl group.

3. The field effect transistor according to claim 1 or 2, **characterized in that** X¹ and X² of Formula (1) are the same and represent a sulfur atom or a selenium atom, and R¹ and R² are each independently a saturated or unsaturated linear, branched or cyclic C1-C4 alkyl group.

4. The field effect transistor according to any one of claims 1 to 3, **characterized in that** X¹ and X² of Formula (1) are the same and represent a sulfur atom or a selenium, atom, and R¹ and R² are each independently a linear C1-C4 alkyl group.

5. The field effect transistor according to any one of claims 1 to 4, **characterized in that** X¹ and X² of Formula (1) are the same and represent a sulfur atom or a selenium atom, and R¹ and R² are the same and represent an unsubstituted linear C1-C4 alkyl group.

6. The field effect transistor according to any one of claims 1 to 5, **characterized in that** X¹ and X² of Formula (1) are the same and represent a sulfur atom or a selenium atom, and R¹ and R² are the same and represent a methyl group or an ethyl group.

7. The field effect transistor according to any one of claims 1 to 6, **characterized in that** X¹ and X² of Formula (1) are the same and represent a sulfur atom or a selenium atom, and R¹ and R² are the same and represent a methyl group.

8. The field effect transistor according to any one of claims 1 to 7, **characterized in that** the semiconductor layer has a single-layer structure.

9. The field effect transistor according to any one of claims 1 to 7, **characterized in that** the semiconductor layer has a single-layer structure formed from a single compound represented by Formula (1).

10. A compound represented by the following Formula (2): 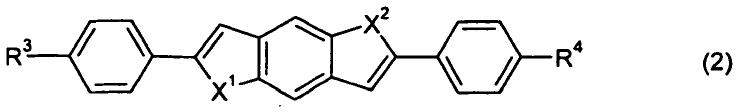 wherein, X¹ and X² each independently represent a sulfur atom, a selenium atom or a tellurium atom, and R³ and R⁴ each independently represent a saturated or unsaturated linear, branched or cyclic C1-C4 alkyl group, with the proviso, that when both of X¹ and X² are a sulfur atom, R³ and R⁴ are not both a methyl group.

11. The compound according to claim 10, wherein X¹ and X² of Formula (2) are the same and represent a sulfur atom or a selenium atom, and R³ and R⁴ are the same and represent an unsubstituted linear C1-C4 alkyl group.

12. The compound according to claim 10, wherein X¹ and X² of Formula (2) are the same and represent a sulfur atom or a selenium atom, and R³ and R⁴ are the same and represent an ethyl group.

13. A semiconductor material **characterized by** comprising at least one compound represented by the following Formula (1): 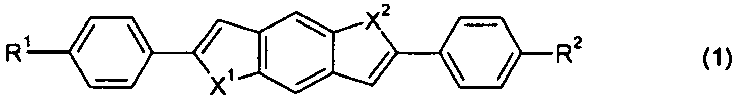 wherein, X¹ and X² each independently represent a sulfur atom, a selenium atom or a tellurium atom, and R¹ and R² each independently represent a saturated or unsaturated linear, branched or cyclic C1-C14 alkyl group, with the proviso that when both of X¹ and X² are a selenium atom, R¹ and R² each independently represent a saturated or unsaturated linear, branched or cyclic C1-C4 alkyl group.

14. A method for manufacturing the field effect transistor according to claim 1, comprising the step of forming, on a substrate, a semiconductor layer formed from at least one compound represented by the following Formula (1): 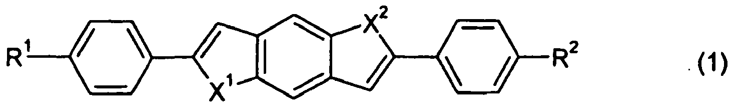 wherein, X¹ and X² each independently represent a sulfur atom, a selenium atom or a tellurium atom and R¹ and R² each independently represent a saturated or unsaturated linear, branched or cyclic C1-C14 alkyl group, with the proviso that when both of X¹ and X² are a selenium atom, R¹ and R² each independently represent a saturated or unsaturated linear, branched or cyclic C1-C4 alkyl group.

15. The method according to claim 14, **characterized in that** X¹ and X² of Formula (1) are the same and represent a sulfur atom or a selenium atom, and R¹ and R² are the same and represent a methyl group.

16. The method according to claim 14 or 15, wherein the semiconductor layer is formed at a substrate temperature within the range of 50 to 80°C.

17. Use of at least one compound represented by the following Formula (1) as a semiconductor material: 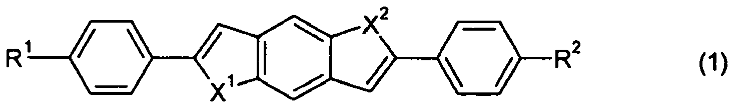 wherein, X¹ and X² each independently represent a sulfur atom, a selenium atom or a tellurium atom, and R¹ and R² each independently represent a saturated or unsaturated linear, branched or cyclic C1-C14 alkyl group, with the proviso that when both of X¹ and X² are a selenium atom, R¹ and R² each independently represent a saturated or unsaturated linear, branched or cyclic C1-C4 alkyl group.

## Patentansprüche

1. . Feldeffekt-Transistor, dadurch charakterisiert, dass er eine Halbleiterschicht aufweiset, die aus mindestens einer Werbindung, dargestellt durch die folgende Formel (1) gebildet ist: 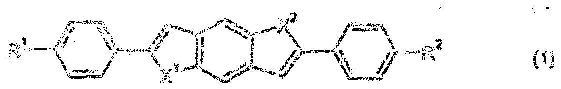 worin X¹ und X² unabhängig voneinander ein Schwefelatom, ein Selenatom oder ein Telluratom darstellen und R¹ und R² jeweils unabhangig voneinander eine gesättigte oder ungesättigte geradkettige, verzweigte oder cyclische (C₁-C₁₄) Alkylgruppe darstellen, mit der Maßgabe, dass, wenn X¹ und X² ein Selenatom darstellen, R¹ und R² unabhängig voreinander eine gesättigte oder ungesättigte geradkettige, verzweigte oder cyclische (C₁-C₄)Alkylgruppe darstellen.

2. . Foldeffekt-Transistor Anspruch 1, dadurch charakterisiert, dass R¹ und R² der Formel (1) jeweils unabhängig voneinander eine gesättigte oder ungesdttigte geradkettige, verzweigte oder cyclische (C₁-C₈)Alkylgruppe darstellen, mit der Maßgabe, wenn X¹ und X² beide ein Selenatom darstellen, R¹ und R² jeweils unabhängig voneinander eine gesättigte oder ungesättigte geradkettige, verzweigte oder cyclische (C₁-C₄)Alkylgruppe darstellen.

3. . Feldeffekt-Transistor gemäß Anspruch 1 oder 2, dadurch charakterisiert, dass X¹ und X² der Formel (1) gleich sind und ein Schwefelatom ein Selenatom darstellen, und R¹ und R² jeweils unabhangig voreinander eine gesättigte oder ungesättigte geradkettige, verzweigte oder cyclische (C₁-C₄)Alkylgruppe darstellen,

4. . Feldeffekt-Transistor gemäß der Ansprüche 1 is 3, dadurch chart dass X¹ und X² der Formel (1) gleich sind und ein schwefelatom oder ein Selenatom darstellen und R¹ und R² jeweils unabhangig voneinander eine geradkettige (C₁-C₄)Alkylgruppe darstellen.

5. . Feldeffekt-Transistor gemäß der Ansprüche 1 bis 4, dadurch charakterisiert, dass X¹ und X² der Formel I (1) gleich sind und ein Schwefelatom oder ein Selenatom darstellen und R¹ und R² gleich sind und unsubstituierte geradkettige (C₁-C₄)Alkylgruppe darstellen.

6. . Feldeffekt-Transistor gemäß einem der Ansprüche 1 is 5, dadurch charakterisiert, dass X¹ und X² der Formel (1) gleich sind und ein Schwefelatom oder ein Selenatom darstellen und R¹ und R² gleich sind und eine Methylgruppe oder eine Ethylgruppe darstellen,

7. . Feldeffekt-Transistor gemäß der Ansprüche 1 bis 6, dadurch charakterisiert, dass X¹ und X² der Formol (1) gleich sind und ein Schwefelatom oder ein Selenatom darstellen und R¹ und R² gleich sind und eine Methylgruppe darstellen.

8. . Feldeffekt-Transistor gemäß einem der Ansprüche 1 bits 7, dadurch charakterisiert, dass die Halbleiterschicht eine einschichtige Struktur aufweist.

9. . Feldeffekt-Transistor gemäß der Ansprüche 1 bis 7, dadurch charakterisiert, dass die Halbleiterschicht eine einschichtig Struktur gebildet aus einer einzigem Verbindung gemäß Formel (1) aufweist,

10. . Verbindung, dargestellt durch dip folgende Formel (2): 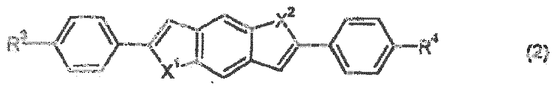 woran X¹ und X² jeweils unabhängig voreinander ein Schwefelatom, ein Selenatom oder ein Telluratom darstellen und R³ und R⁴ jeweils unabhängig voneinander eine gesättigte oder ungesättigte geradkettige, verzweigte oder cyclische (C₁-C₄)Alkylgruppe darstellen, mit der Maßgabe, dass, wenn X¹ und X² beide ein Schwefelatom sind, R³ und R⁴ nicht belde eine Methylgruppe darstellen,

11. . Verbindung gemäß Anspruch 10, worin X¹ und X² der Formel (2) gleich sind und ein Schwefelatom oder ein Selenatom darstellen und R³ and R⁴ gleich sind und eine unsubstituierte geradkettige (C₁-C₄)Alkylgruppe darstellen.

12. . Verbindung gemäß Anspruch 10, worin X¹ und X² der Formel (2) gleich sind und ein Schwefelatom oder ein Selenatom darstellen und R³ und R⁴ gleich sind und eine Ethylgruppe darstellen

13. . Halbleiter-Material, dadurch charakterisiert, dass es mindestens eine Verbindung, dargestellt durch dip folgende Formel (1) enthält: 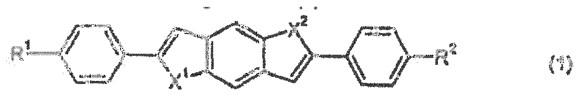 woran X¹ und X² jeweils unabhängig voneinander ein Schwefelatom, ein Selenatom oder ein Telluratom darstellen und R¹ und R² jeweils unabhängig voneinander eine gesättigte oder ungesättigte geradkettige, verzweigte oder cyclische (C₁-C₁₄)Alkylgruppe darstellen, mit der Maßgabe, dass, wenn X¹ und X² beide ein Selenatom darstellen, R¹ und R² jeweils unabhängig voreinander eine gesättigte oder ungesättigte geradkettige, verzweigte oder cyclische (C₁-C₄)Alkylgruppe darstellen,

14. . Verfahren zur Herstellung eines Feldeffekt-Transistors gefäß Anspruch 1, umfassend den Schritt der Bildung einer Halbleiterschicht, gebildet aus mindestens einer Verbindung dargestellt durch die folgende Formel (1), auf einem Substrat: 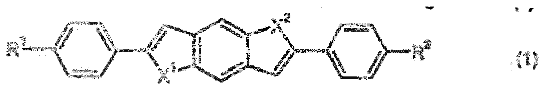 woran X¹ und X² jeweils unabhängig voneinander ein Schwefelatom, ein Selenatom oder ein Telluratom darstellen und R¹ und R² jeweils unabhängig voneinander eine gesättigte oder ungesättigte geradkettige, verzweigte oder cyclische (C₁-C₁₄)Alkylgruppe darstellen, mit der Maßgabe, dass, wenn X¹ und X² beide ein Selenatom darstellen, R¹ und R² jeweils unabhängig voneinander eine gesättigte oder ungesättigte geradkettige, verzweigte oder cyclische (C₁-C₄)Alkylgruppe darstellen.

15. . Verfahren Anspruch 14, dadurch charakterisiert, **dass X¹** und X² der Formel (1) gleich sind und ein Schwefelatom oder ein Selenatom und R¹ und R² gleich sin und eine Methylgruppe darstellen.

16. . Verfahren gemäß Anspruch 14 oder 15, worin die Halbleiterschicht bel einer Substrat-Temperatur zwischen 50 und 80°C gebildet ist,

17. . Verwendung mindestens Verbindung, dargestellt durch die folgende Formol (1), als Halbleiter-Material: 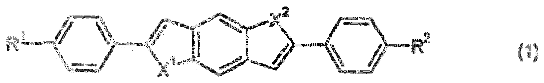 woran X¹ und X² jeweils unabhängig voneinander ein Schwefelatom, ein Selenatom oder ein Telluratom darstellen und R¹ und R² jeweils unabhangig voreinander eine gesättigte oder ungesättigte geradkettige, verzweigte oder cyclische (C₁-C₁₄)Alkylgruppe darstellen, mit der Maßgabe, dass, wenn X¹ und X² beide ein Selenatom darstellen, R¹ und R² jeweils unabhängig voneinander eine gesättigte oder ungesättigte geradkettige, verzweigte oder cyclische (C₁-C₄)Alkylgruppe darstellen,

## Revendications

1. Transistor à effet de champ **caractérisé par le fait qu'**il possède une couche semi-conductrice formée par au moins un composé représenté par la formule (1) suivants : 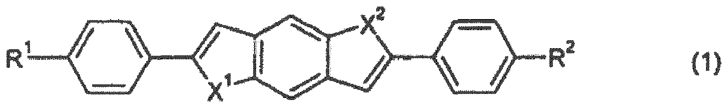 dans laquelle chacun de X¹ et X² représente indépendamment un atome de soufre, un atome de sélénium ou un atome de tellure et chacun de R¹ et R² représente indépendamment un groupe alkyle linéaire, ramifié ou cyclique, saturé ou insaturé en C1-C14, à la condition que lorsque X¹ et X² sont chacun un atome de sélénium, chacun de R¹ et R² représente indépendamment un groupe alkyle linéaire, ramifié ou cyclique, saturé ou insaturé en C1-C4.

2. Transistor à effet de champ selon la revue 1, **caractérisé en ce que** chacun de R¹ et R² de la formule (1) est indépendamment un groupe alkyle linéaire, ramifié ou cyclique, saturé ou insaturé en C1-C8, à la condition que lorsque X¹ et X² sont chacun un atome de sélénium, chacun de R¹ et R² représente indépendamment un groupe alkyle linéaire, ramifié ou cyclique, saturé ou insaturé en C1-C4.

3. Transistor à effet de champ selon l'une des revendications 1 ou 2, **caractérisé en ce que** et X² de la formule (1) sont les mêmes et représentent un atome de soufre ou un atome de sélénium et chacun de R¹ et R² sont indépendamment un groupe alkyle linéaire, ramifié ou cyclique, saturé ou insaturé en C1-C4.

4. Transistor à effet de champ selon l'une des revendications 1 à 3, **caractérisé en ce que** X¹ et X² de la formule (1) sont les mêmes et représentent un atome de soufre ou un atome de sélénium et chacun de R¹ et R² est indépendamment un groupe alkyle linéaire en C1-C4.

5. Transistor à effet de champ selon l'une des revendications 1 à 4, **caractérisé en ce que** X¹ et X² de la formule (1) sont les mêmes et représentent un atome de soufre ou un atome de sélénium et R¹ et R² sont les mêmes et représentent un groupe alkyle linéaire non substitué en C1-C4.

6. Transistor à effet de champ selon l'une des revendications 1 à 5, **caractérisé en ce que** X¹ et X² de la formule (1) sont les mêmes et représentent un atome de soufre ou un atome de sélénium et R¹ et R² sont les mêmes et représentent un groupe méthyle ou un groupe éthyle.

7. Transistor à effet de champ selon l'une de revendications 1 à 6, **caractérisé en ce que** X¹ et X² de la formule (1) sont les mêmes et représentent un atome de soufre ou un atome de sélénium et R¹ et R² sont les mêmes et représentent un groupe méthyle,

8. Transistor à effet de champ selon l'une des revendications 1 à 7, chartérisé en ce que la couche semi-conductrice présente une structure monocouche.

9. Transistor à effet de champ selon l'une des revendications 1 à 7, **caractérisé en ce que** la couche semi-conductrice présente une structure monocouche formée par un seul composé représenté par la formule (1).

10. Composé par la formule (2) suivante ; 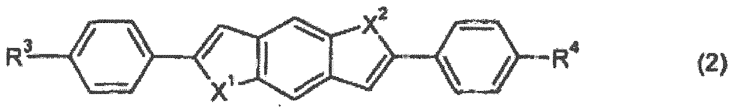 dans laquelle chacun de X¹ et X² représente indépendamment un atome de soufre, un de sélénium ou un atome de tellure et chacun de R³ et R⁴ représente indépendamment un groupe alkyle linéaire, ramifié ou cyclique, saturé ou insaturé en C1-C4, à la condition que lorsque X¹ et X² sont chacun un atome de soufre, R³ et R⁴ ne soient pas tous deux un groupe méthyle.

11. Composé selon la revendication 10, dans lequel X¹ et X² de la formule (2) sont les mêmes et représentent un atome de soufre ou un atome de sélénium et R³ et R⁴ sont les mêmes et représentent un groupe alkyle linéaire non substitué en C1-C4.

12. Composé selon la revendication 10, dans lequel X¹ et X² de la formule (2) sont les mêmes et représentent un atome de soufre ou un atome de sélénium et R³ et R⁴ sont les mêmes et représentent un groupe éthyle.

13. Matériau semi-conducteur **caractérisé par le fait qu'**il comprend au moins un composé par la formule (1) suivante : 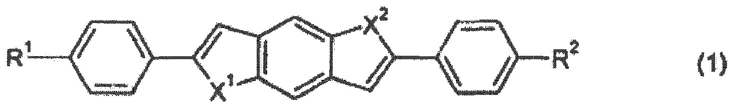 dans laquelle chacun de X¹ et X² représente indépendamment un atome de soufre, un atome de sélénium ou un atome de tellure et chacun de R¹ et R² représente indépendamment un groupe alkyle linéaire, ramifié ou cyclique, saturé ou insaturé en C1-C14, à la condition que lorsque X¹ et X² sont chacun un atome de sélénium, chacun de R¹ et R² représente indépendamment un groupe alkyle linéaire, ramifié ou cyclique, saturé ou insaturé en C1-C4.

14. Procédé de production d'un transistor à effet de champ selon la revendication 1, comprenant l'étape de formation d'une couche semi-conductrice formée par au moins un composé représenté par la formule (1) suivante sur un support : 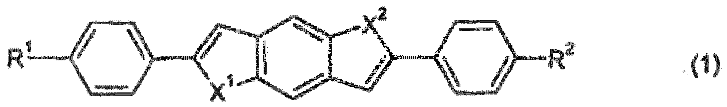 dans laquelle chacun de X¹ et X² représente indépendamment un atome de soufre, un atome de sélénium ou un atome de tellure et chacun de R¹ et R² représente indépendamment un groupe alkyle linéaire, ramifié ou cyclique, saturé ou insaturé en C1-C14, à la condition que lorsque X¹ et X² sont chacun un atome de sélénium, chacun de R¹ et R² représente indépendamment un groupe alkyle linéaire, ramifié ou cyclique, saturé ou insaturé en C1-C4.

15. Procédé selon la revendication 14, **caractérisé en ce que** X¹ et X² de la formule (1) sont les mêmes et représentent un atome de soufre ou un atome de sélénium et R¹ et R² sont les mêmes et représentent un groupe méthyle.

16. Procédé selon l'une des revendications 14 ou 15, dans lequel la couche semi-conductrice est à une température du support de 50 à 80 °C.

17. Utilisation d'au moins un composé représenté par la formule (1) suivante en tant qu' un matériau semi-conducteur : 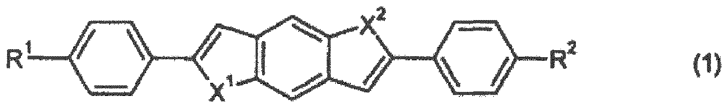 dans laquelle chacun de X¹ et X² représente indépendamment un atome de soufre, un atome de sélénium ou un atome de tellure et chacun de R¹ et R² représente indépendamment un groupe alkyle linéaire, ramifié ou cyclique, saturé ou insaturé en C1-C14, à la condition que lorsque X¹ et X² sont chacun un atome de sélénium, chacun de R¹ et R² représente indépendamment un groupe alkyle linéaire, ramifié ou cyclique, saturé ou insaturé en C1-C4.
